# FASCICULE DE BREVET EUROPEEN

(11) **EP 1 332 750 B1**
(45) Date de publication et mention de la délivrance du brevet: **06.09.2006**
(21) Numéro de dépôt: 03290211.6
(22) Date de dépôt: 28.01.2003
(51) Int. Cl.: A61K 8/03, A61K 8/73, A61Q 3/02

(54) **Vernis à ongles multiphase**
Mehrphasiger Nagellack
Mutiphasic nail varnish

(30) Priorité: 30.01.2002 FR 0201077
(43) Date de publication de la demande: 06.08.2003
(73) Titulaire: Fiabila, 28130 Maintenon (FR)
(72) Inventeur: Malnau, Alain, 27350 Routot (FR); Martinez, Francisco, 28000 Chartres (FR)
(74) Mandataire: Laget, Jean-Loup

(56) Documents cités:
- EP-A- 0 847 751
- FR-A- 2 819 176
- US-A- 5 370 866

## Description

La présente invention concerne le domaine des vernis à ongles, et plus particulièrement un vernis à ongles multiphase.

Typiquement, une composition de vernis à ongles comporte un polymère filmogène principal, généralement cellulosique, des polymères secondaires comme les résines polyesters, acryliques ou de condensation avec le tosylamide, pour améliorer les caractéristiques physiques du film, un ou plusieurs plastifiants pour rendre le polymère principal plus flexible et des solvants. Ce mélange de base peut également contenir des matières colorantes, des agents de suspension et des additifs tels que des agents anti-UV, des agents mouillants, des agents de glissance, des agents hydratants, des parfums, etc...

Pour que ces vernis à ongles aient de bonnes propriétés cosmétiques (brillant, adhérence, durabilité, ...), il était jusqu'à maintenant très important que les polymères soient compatibles entre eux, afin d'obtenir un mélange homogène et stable dans le temps sans déphasage et sans séparation, le déphasage étant considéré comme un critère de non-qualité.

Cependant, l'inventeur a récemment mis au point une composition de vernis à ongles, se présentant au repos sous la forme d'au moins deux phases stables qui, après agitation, redevient homogène et peut être appliquée très facilement sur les ongles. Après séchage, ce vernis possède les propriétés d'un vernis de composition classique.

La composition selon l'invention est une composition de vernis à ongles, sans eau, comprenant
- un polymère filmogène cellulosique,
- un plastifiant et
- un ou des solvant(s),
caractérisée en ce qu'elle renferme de 20 à 200 % en poids, par rapport au polymère cellulosique, d'au moins un deuxième polymère filmogène incluant le polyvinyl butyral, partiellement insoluble dans ledit polymère cellulosique, provoquant une démixtion d'au moins deux phases liquides stables et distinctes au repos.

De préférence, le polymère cellulosique est la nitrocellulose ou l'acétobutyrate de cellulose ou un mélange de ceux-ci.

Pour obtenir ce déphasage stable, l'inventeur a trouvé que parmi les polymères filmogènes partiellement incompatibles avec le ou les polymère(s) filmogène(s) cellulosique(s) en solution dans un mélange de solvants, sont ceux incluants le polyvinyl butyral.

Le polyvinylbutyral a déjà été utilisé dans diverses compositions pour vernis en vue :
- soit de remplacer totalement la nitrocellulose (cf. US-A-4 283 324),
- soit de la remplacer partiellement, mais uniquement dans des conditions où il ne perturbait pas l'homogénéité du vernis (dans des proportions où il était «compatible» c'est-à-dire «soluble», dans les autres résines, notamment la nitrocellulose) : **par exemple en faibles quantités (US-A-5 370 866) en présence d'agent épaississant, ou dans des proportions très importantes comprises entre 20 et 30 fois la quantité de nitrocellulose (FR-A-2 819 176), en présence de bentone.**

Cependant, dans le cas présent, ces résines vinyliques, et en particulier le polyvinylbutyral permettent d'une part, lorsque la composition est au repos prolongé, d'obtenir une démixtion, et d'autre part, dès que l'on agite cette composition, celle-ci présente un aspect homogène et donne également sur l'ongle une apparence tout à fait uniforme.

De manière avantageuse, la concentration de ce deuxième polymère incluant le polyvinyl butyral est supérieure à environ 3 % en poids de la composition totale de vernis.

Les proportions préférées en deuxième polymère filmogène représentent de 80 à 200 %, avantageusement de 120 à 180 % en poids du polymère cellulosique.

Le solvant (ou le mélange de solvants) est un solvant à la fois du premier polymère filmogène (cellulosique), et du deuxième polymère partiellement incompatible avec le premier. Il permet aussi de régler la démixtion entre les deux solutions de polymère.

Le solvant ou le mélange de solvants est choisi parmi les composés aliphatiques, tels que les acétates, les cétones, les alcools, les alcanes ou un mélange de ceux-ci, et plus particulièrement parmi l'acétate d'éthyle, l'acétate de butyle, l'acétate de propyle, l'acétate d'isobutyle, l'éthanol, le propanol, l'isopropanol, le butanol, la méthyléthylcétone, la méthylisobutylcétone, l'heptane et l'hexane, et des mélanges de ceux-ci.

On obtient alors deux phases parfaitement limpides et stables dans le temps. La hauteur de ces phases varie avec les polymères, leurs proportions et avec les solvants utilisés. Après agitation, ces deux phases donnent un mélange homogène qui a les mêmes propriétés qu'une composition classique.

Un tel solvant ou mélange de solvants permet, lorsque le vernis est agité, de favoriser une très fine émulsion des polymères filmogènes entre eux, et d'empêcher, lors du séchage, que l'un de ces polymères ne précipite, ce qui nuirait à une bonne adhérence du vernis sur l'ongle.

La composition selon l'invention peut aussi renfermer une ou plusieurs matières colorantes choisies parmi les pigments, les colorants solubles, notamment les colorants classiques utilisés en cosmétique, et les particules dites décoratives, telles que nacres ou paillettes.

L'inventeur a également découvert que les matières colorantes dispersées dans un des polymères, comme les pigments, restaient exclusivement dans une des phases, celle renfermant le polymère filmogène cellulosique. Par contre, les colorants en solution se répartissent dans les deux phases de manière quasi équivalente.

Ce phénomène permet d'obtenir une composition de vernis à ongles multiphase, présentant au repos au moins deux phases stables, pouvant être de couleurs différentes, notamment si la phase cellulosique contient un pigment.

La composition peut également contenir des particules colorées qui, après sédimentation, donnent naissance à une troisième couche de couleur, moins importante, dans le fond du flacon.

On obtient ainsi dans le flacon, au repos, un vernis à ongles en plusieurs couches, colorées au moyen des matières colorantes qui sont soit en suspension, soit dispersées dans le polymère cellulosique, soit en solution, teintant respectivement le fond du flacon, la phase inférieure ou la phase supérieure du vernis.

Après homogénéisation, la composition selon l'invention décrite ci-dessus peut être utilisée pour une application sur les ongles comme vernis à ongles seul, comme vernis de finition ou comme couche intermédiaire.

La présente invention est illustrée par les exemples de réalisations de vernis à ongles multiphases ci-après (les indications chiffrées sont des pourcentages en poids de la composition totale).

### Exemple 1

Les constituants suivants sont mélangés dans les conditions habituelles de préparation des vernis à ongles.

| | |
|---|---|
| Nitrocellulose 70 % dans isopropanol | 7,6 |
| Plastifiant | 2,2 |
| Résine acrylique thermoplastique | 4,9 |
| Polyvinylbutyral | 8,3 |
| Acétate d'éthyle 44 | |
| Acétate de butyle 28 | |
| Heptane | 5 |

Le polyvinylbutyral est partiellement incompatible avec la nitrocellulose dans ce mélange de solvants. Au repos cette composition subit une démixtion en deux phases, de volumes équivalents, parfaitement limpides. La phase inférieure est riche en nitrocellulose et la phase supérieure est riche en résine vinylique.

Si on ajoute à cette composition un colorant soluble dans les solvants cités, la couleur se répartit uniformément dans les deux phases.

### Exemple 2

Dans la composition selon l'exemple 1, on ajoute une dispersion de Bentone®, de pigment rouge, un pigment sous forme de particules et une solution de colorant dans les proportions indiquées ci-dessous. Les dispersions de Bentone et de pigment coloré sont préalablement broyées dans la solution de nitrocellulose + plastifiant.

| | |
|---|---|
| Nitrocellulose 70 % dans isopropanol | 7,4 |
| Plastifiant | 2,1 |
| Résine acrylique thermoplastique | 4,7 |
| Polyvinylbutyral | 8 |
| Acétate d'éthyle | 43 |
| Acétate de butyle | 27 |
| Bentone | 0,3 |
| Red 7 Ca lake | 0,3 |
| Mica titane / Oxyde de fer jaune | 2 |
| Solution de colorant bleu | 0,5 |

Comme dans l'exemple précédent, on observe au repos deux phases d'égales proportions volumiques.

Comme la composition n'est pas fortement «gélifiée», les particules de mica/titane/oxyde de fer jaune sédimentent, et forment une troisième couche au fond du flacon.

Le pigment rouge se répartit d'une façon uniforme, exclusivement dans la phase liquide inférieure riche en nitrocellulose.

Le colorant bleu se répartit dans les trois phases, mais comme son pouvoir colorant est faible, il n'est visible que dans la phase liquide supérieure, transparente.

On obtient donc dans le flacon au repos, un vernis en trois phases : un petit dépôt jaune solide au fond du flacon, puis une phase rouge et une phase supérieure bleue.

Après homogénéisation par agitation, on obtient dans le flacon une composition rouge avec des reflets oranges tout comme le film obtenu sur l'ongle après application.

Le temps de démixtion (déphasage) étant de plusieurs heures, la composition reste homogène pendant la durée de son application sur l'ongle et de son séchage.

Cette composition a, sur l'ongle, les mêmes propriétés qu'un vernis traditionnel.

## Revendications

1. Composition de vernis à ongles, sans eau, comprenant
- un polymère filmogène cellulosique,
- un plastifiant et
- un ou des solvant(s),
**caractérisée en ce qu'**elle renferme de 20 à 200 % en poids, par rapport au polymère cellulosique, d'au moins un deuxième polymère filmogène, **incluant le polyvinyl butyral,** partiellement insoluble dans ledit polymère cellulosique, provoquant une démixtion d'au moins deux phases liquides stables et distinctes au repos.

2. Composition selon la revendication 2, **caractérisée en ce que** le polymère cellulosique est la nitrocellulose ou l'acétobutyrate de cellulose ou un mélange de ceux-ci.

3. Composition selon l'une quelconque des revendications 1 ou 2, **caractérisée en ce que** la quantité du deuxième polymère est supérieure à environ 3 % en poids de la composition totale de vernis.

4. Composition selon l'une quelconque des revendications précédentes, **caractérisée en ce que** le deuxième polymère filmogène représente de 80 à 200 % en poids du polymère cellulosique.

5. Composition selon la revendication 4, **caractérisée en ce que** le deuxième polymère filmogène représente de 120 à 180 % en poids du polymère cellulosique.

6. Composition selon l'une quelconque des revendications précédentes, **caractérisée en ce que** le ou les solvant(s) des polymères sont choisis parmi les composés aliphatiques, tels que les acétates, les cétones, les alcools, les alcanes ou un mélange de ceux-ci.

7. Composition selon la revendication 6, **caractérisée en ce que** le(s) solvant(s) est(sont) choisi(s) parmi l'acétate d'éthyle, l'acétate de butyle, l'acétate de propyle, l'acétate d'isobutyle, l'éthanol, le propanol, l'isopropanol, le butanol, la méthyléthylcétone, la méthylisobutylcétone, l'heptane et l'hexane, et des mélanges de ceux-ci.

8. Composition selon l'une quelconque des revendications précédentes, **caractérisée en ce qu'**elle renferme une ou plusieurs matières colorantes choisies parmi les pigments, les colorants solubles, les particules décoratives, telles que nacres, ou paillettes.

9. Utilisation de la composition selon l'une des revendications 1 à 8, après homogénéisation, pour une application sur les ongles comme vernis à ongle seul, comme vernis de finition ou comme couche intermédiaire.

## Claims

1. An anhydrous nail varnish composition, comprising
- a film-forming cellulose polymer,
- a plasticiser and
- one or more solvent(s),
**characterised in that** it contains from 20 to 200% by weight, relative to the cellulose polymer, of at least one second film-forming polymer, including polyvinyl butyral, which is partially insoluble in said cellulose polymer, causing demixing of at least two stable, distinct liquid phases at rest.

2. A composition according to Claim 2, **characterised in that** the cellulose polymer is nitrocellulose or cellulose acetobutyrate or a mixture thereof.

3. A composition according to either one of Claims 1 or 2, **characterised in that** the quantity of the second polymer is greater than approximately 3% by weight of the total varnish composition.

4. A composition according to any one of the preceding claims, **characterised in that** the second film-forming polymer represents from 80 to 200% by weight of the cellulose polymer.

5. A composition according to Claim 4, **characterised in that** the second film-forming polymer represents from 120 to 180% by weight of the cellulose polymer.

6. A composition according to any one of the preceding claims, **characterised in that** the solvent(s) for the polymers is(are) selected from among aliphatic compounds, such as acetates, ketones, alcohols, alkanes or a mixture thereof.

7. A composition according to Claim 6, **characterised in that** the solvent(s) is(are) selected from among ethyl acetate, butyl acetate, propyl acetate, isobutyl acetate, ethanol, propanol, isopropanol, butanol, methyl ethyl ketone, methyl isobutyl ketone, heptane and hexane, and mixtures thereof.

8. A composition according to any one of the preceding claims, **characterised in that** it includes one or more colorants selected from among pigments, soluble dyes, decorative particles, such as pearlescent pigments, or flakes.

9. The use of the composition according to one of Claims 1 to 8, after homogenisation, for application to nails such as a single nail varnish, a top coat or an intermediate layer.

## Patentansprüche

1. Wasserfreie Nagellackzusammensetzung umfassend
- ein filmbildendes, cellulosehaltiges Polymer,
- einen Weichmacher und
- ein oder mehrere Lösungsmittel,
**dadurch gekennzeichnet, daß** sie, bezogen auf das cellulosehaltige Polymer, 20 bis 200 Gew.-% mindestens eines zweiten filmbildenden Polymers, einschließlich Polyvinylbutyral, enthält, das in dem cellulosehaltigen Polymer teilweise unlöslich ist, was eine Entmischung mindestens zweier im Ruhezustand stabiler und unterschiedlicher flüssiger Phasen bewirkt.

2. Zusammensetzung nach Anspruch 2, **dadurch gekennzeichnet, daß** das cellulosehaltige Polymer Nitrocellulose oder Celluloseacetobutyrat oder ein Gemisch davon ist.

3. Zusammensetzung nach einem der Ansprüche 1 oder 2, **dadurch gekennzeichnet, daß** die Menge des zweiten Polymers mehr als etwa 3 Gew.-% der Gesamtlackzusammensetzung beträgt.

4. Zusammensetzung nach einem der vorhergehenden Ansprüche, **dadurch gekennzeichnet, daß** das zweite filmbildende Polymer 80 bis 200 Gew.-% des cellulosehaltigen Polymers ausmacht.

5. Zusammensetzung nach Anspruch 4, **dadurch gekennzeichnet, daß** das zweite filmbildende Polymer 120 bis 180 Gew.-% des cellulosehaltigen Polymers ausmacht.

6. Zusammensetzung nach einem der vorhergehenden Ansprüche, **dadurch gekennzeichnet, daß** das oder die Lösungsmittel der Polymere unter aliphatischen Verbindungen, wie Acetaten, Ketonen, Alkoholen, Alkanen, oder einem Gemisch davon ausgewählt ist (sind).

7. Zusammensetzung nach Anspruch 6, **dadurch gekennzeichnet, daß** das oder die Lösungsmittel aus Ethylacetat, Butylacetat, Propylacetat, Isobutylacetat, Ethanol, Propanol, Isopropanol, Butanol, Methylethylketon, Methylisobutylketon, Heptan und Hexan und Gemischen davon ausgewählt ist (sind).

8. Zusammensetzung nach einem der vorhergehenden Ansprüche, **dadurch gekennzeichnet, daß** sie ein Färbematerial oder mehrere Färbematerialien enthält, die aus Pigmenten, löslichen Farbstoffen, dekorativen Teilchen, wie Perlmutt oder Pailletten, ausgewählt sind.

9. Verwendung der Zusammensetzung nach einem der Ansprüche 1 bis 8 nach Homogenisierung, zum Aufbringen auf die Nägel als alleiniger Nagellack, als Oberflächenlack oder als Zwischenschicht.
